# EUROPEAN PATENT APPLICATION

(11) **EP 2 384 742 A1**
(43) Date of publication of application: **09.11.2011**
(21) Application number: 10004631.7
(22) Date of filing: 03.05.2010
(51) Int. Cl.: A61K 9/00, A61K 9/20, A61K 47/26, A61K 47/36

(54) **Pharmaceutical excipient, method for its preparation and use thereof**

(71) Applicant: The Jordanian Pharmaceutical Manufacturing Co., 11710 Naor (JO)
(72) Inventor: Daraghmenh, Nidal H., 11710 Naor (JO); Al Omari, Mahmoud M., 11710 Naor (JO); Badwan, Adnan A., 11710 Naor (JO)
(74) Representative: Winkler, Andreas Fritz Ernst

(57) **Abstract**

The present invention relates to a pharmaceutical excipient comprising a mixture of sugar alcohol and chitin and/or chitin derivatives, a method for its preparation and use thereof.

## Description

The invention relates to a pharmaceutical excipient, a method for its preparation and use thereof.

Chitin is a long-chain polymer of N-acetylglucosamine, a derivative of glucose, and it is found in many places throughout the natural world. It is the main component of the cell walls of fungi, the exoskeletons of arthropods, such as crustaceans (like crab, lobster and shrimp) and insects, including ants, beetles and butterflies, the radula of molluscs and the beaks of the cephalopods, including squid and octopi. Chitin is a polysaccharide; it is synthesized from units of N-acetylglucosamine. These units form covalent β-1,4-linkages (similar to the linkages between glucose units to forming cellulose). Chitin may therefore be described as cellulose with one hydroxyl group on each monomer substituted with an acetylamine group represented as follows:

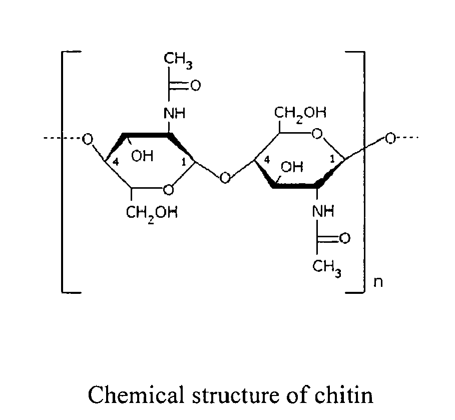

Next to cellulose, it is the most abundant polysaccharide on earth and for this reason there is continuing interest in finding new commercial uses for what is now essentially a waste product from the crab and shrimp industry.

Chitosan is a polysaccharide [(1-4)-2-amino-2-deoxy-β-D-glucan]. It is prepared by alkaline deacetylation of chitin, and in practice this process is not fully completed; therefore, chitosan with different percentages of deacetylation can be formed depending on the condition of the deacetylation (acceptable > 85%). It is used in pharmaceutical formulations as a filler or binder and in modified release dosage forms.

A sugar alcohol (also known as a polyol, polyhydric alcohol, or polyalcohol) is a hydrogenated form of a carbohydrate, whose carbonyl group (aldehyde or ketone, reducing sugar) has been reduced to a primary or secondary hydroxyl group. Its general formula is H(HCHO)n+1H, whereas that of the sugar is H(HCHO)nHCO. Mannitol is one example of these polyalcohols. It is found in abundance in nature, particularly in exudates from trees, and in marine algae and fresh mushrooms. It is an isomer of sorbitol and is typically produced, currently, by the hydrogenation of speciality glucose syrups. It is widely used in the food and pharmaceutical industries because of its unique functional properties. It is about 50% as sweet as sucrose and has a desirable "cooling" effect often used to mask bitter tastes. Unlike sorbitol, a polyol often used for its humectant properties, mannitol is non-hygroscopic (does not pick up moisture), in addition it has a pleasant taste, is very stable to moisture pickup and does not discolour at high temperature, which makes mannitol ideal for use in pharmaceutical tablet formulations.

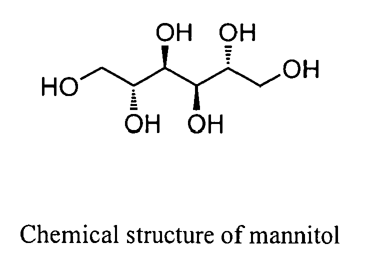

To prepare a tablet dosage form, many additives are necessary to produce units with acceptable physicochemical properties. The additives may include filler, binder, glidant, and lubricant. It is necessary that materials to be compressed into tablets possess certain physical properties. Among these, the materials must be free flowing, cohesive, and lubricated. To allow the active pharmaceutical ingredient release from the solid dosage forms, it is necessary to use a disintegrant, which is added to a tablet to facilitate its break up or disintegration after administration. Starches are the most widely used tablet disintegrants. In addition to starches, a large variety of materials have been used and reported to be effective as tablet disintegrants (e. g. veegum HV, methylcellulose, agar, bentonite, cellulose product, and algenic acid). Notwithstanding the asserted efficacy of these materials as tablet components, several disadvantages are related to these additives, such as high cost, merely moderate efficiency, long processes for preparing the excipients, and possible adverse effects upon the pharmaceutical active ingredients and potential deterioration resulting in decreased utility after long periods of storage.

Current technologies involved in many patents as well as existing commercial fast-disintegrating tablets utilize complicated processing techniques such as freeze-drying, spray-drying, molding and sublimation or use of specialized excipients such as effervescent couple, highly micronized agents or the likes.

Freeze-drying is one common process for producing many commercial fast-dissolving tablets wherein a cake or wafer is prepared by freeze-drying a solution or suspension of the medicament and suitable excipients in water or other solvents. Such systems dissolve very rapidly on the tongue, due to their high affinity for moisture and a very high porosity.

U.S. Pat. No. 5,298,261 discloses freeze-drying a slurry or paste comprising an active ingredient and excipients placed in blister packets. PCT application WO 97/36879 discloses vacuum drying, at room temperature or a slightly elevated temperature, of a suspension including the active drug, a sugar alcohol, PEG 6000, talc, sweeteners and flavors, in preformed blisters. However, the freeze-drying process suffers from several disadvantages. The primary disadvantage is that solutions employed for freeze-drying are aqueous and, therefore, not suited for water sensitive medicaments. Freeze-drying is also limited to low dose actives. The process itself is typically laborious, costly and time-consuming. Finally, the resultant dosage forms, in addition to being hygroscopic, tend to be very soft, and therefore require special moisture-resistant and impact-resistant packaging and careful handling.

U.S. Pat. No. 2009087485 discloses spray-drying slurry of calcium silicate and carbohydrate, followed by blending with active ingredient(s), co-filler, sweeteners, flavors and lubricant. However, the disadvantage of spray-drying is that the process itself is typically laborious, costly and time-consuming. In addition, the use of suspended particles of non-inert calcium silicate at elevated temperature may cause difficulties during spray-drying as the process is particle size dependant and subsequently leads to blockage of the spray nozzles.

U.S. Pat. No. 5,464,632 claims the use of high levels of disintegrants, such as 16% starch 1500 and 13.3% crospovidone, for a disintegration time of 35 seconds to 45 seconds. However, such tablets have a chalky or dry feel when placed in the mouth.

U.S. Pat. No. 5,178,878 discloses a rapidly dissolving oral formulation that requires an extra-granular microparticulate active in conjunction with an effervescent agent incorporated into a tableted matrix in order to achieve rapid oral disintegration. Many fast-dissolving tablets are also formulated by the inclusion of effervescent compounds. U.S.

Pat. No. 5,178,878 and WO 91/04757 disclose the addition of an effervescent couple (such as sodium bicarbonate and citric acid) to a tablet. Exposure of such tablet to moisture results in contact and chemical reaction between the effervescent couple which leads to gas production and tablet disintegration. However, tablets, which include effervescent pairs, are highly sensitive to moisture and require a specific, very costly plant including special handling equipment, controlled-humidity environments, as well as special moisture resistant packaging. Such preparations have an unpleasant mouth feel.

Another orally disintegrating technique is spray drying technology as explained in U.S. Pat. Nos. 5,958,471 and 6,165,511, which includes preparing an aqueous solution of more than 80% of one or more non-hygroscopic polyols, and spraying the resulting mixture into an air stream. The resulting composition of the spray-drying process contains a filamentous structure. Similarly PCT application WO 03/051338A1 relates to a method for producing a directly compressible and highly compactible composition by cospray drying of mannitol and sorbitol solution resulting in nonfilamentous microstructure. Both these patents describe use of highly concentrated aqueous solutions, which are to be maintained and sprayed at high temperature, thus demanding special equipment.

Another approach to develop orally disintegrating dosage form involves optimal selection of excipients, which would result in desired disintegration time. These are typically compressed dosage forms. EP 1145711 describes the preparation of flash-melt dosage forms that disintegrate in the mouth in less than 25 second. They consist of granules composed of a superdisintegrant (4-8%), a dispersing agent such as calcium silicate (20-70%), a distributing agent selected from amorphous silica, fumed silica, diatomaceous earth, talc, kaolin, magnesium aluminum trisilicate, and a binder (10 to 50% by weight). Although a larger amount of binder may produce stronger tablets, the disintegration times tend to increase. To counter this, a large amount of dispersing and distributing agent is included in the formulation, which increases the weight of the tablet. This may increase the cost of the formulation.

PCT application WO 03/045844A1 relates to synthetic calcium metasilicate, which when incorporated in a solid product, significantly increases the disintegration rate of the formed product when contacted by a substantially aqueous environment. The reduction in disintegration time with calcium silicate is more pronounced with immediate release tablets, as tablets prepared with calcium silicate have lower porosity. Further, the use of calcium silicate with conventional equipments leads to discoloration of the final dosage form due to interaction of calcium silicate with some metals, as well as reaction with many active pharmaceutical agents (hereinafter the "API".) Calcium silicate, due to its hydrophobic and static nature, results in blends with very poor flow properties causing weight and content variation during compression into tablets. Further, it also imparts a chalky taste to the dosage form.

In general, there are numerous other examples of specific formulations that utilize one or more of the techniques or mechanisms discussed above. Majority of these techniques possess one or more of the above enumerated disadvantages to some extent such as tedious and complex method of manufacturing, special packaging and storage requirements, high cost, limitation on drug load etc. Thus, there continues to be a need for a formulation that mitigates or eliminates these disadvantages. The desired features of such dosage form include quick disintegrability in an oral cavity, a pleasant mouth feel and optimal mechanical strength even in storage under a humidifying condition.

Chitin and chitin derivatives suffer from low compressibility and low compactibility that often hampers their application. Surprisingly, the difficulties of the prior art may be overcome by the pharmaceutical excipient according to the present invention, which provides properties of improved flowability, high compactibility and compressibility as well as fast disintegration. In addition, the co-processed excipient according to the invention is not sensitive to the presence of sufficient amount of an effective lubricant, such as metal stearates.

Sugar alcohol suffers from low compressibility and low compactibility that often hampers their application. Surprisingly, the difficulties of the prior art may be overcome by the pharmaceutical excipient according to the present invention, which provides properties of improved flowability, high compactibility and compressibility as well as fast disintegration.

It was surprisingly found that composite of various proportions made by co-processing of a mixture of sugar alcohol and chitin/or a chitin derivative provides excipients that rapidly disintegrates in the mouth or used as super disintegrant and filler with excellent binding properties.

More specifically, it has been discovered that a composite excipient formed by a mixture of chitin/or a chitin derivative and sugar alcohol exhibits greatly improved flow properties. Tablets made with this improved composite excipient exhibit low friability, low ejection forces and hardness sufficient to be processed in high speed tableting machines, while retaining rapid disintegration or dissolution properties.

It is an object of the present invention to overcome the disadvantages of the prior art and to provide an improved pharmaceutical excipient. Further, a method for its preparation and use shall therefore be provided.

The first object is achieved by the pharmaceutical excipient comprising chitin and/or a chitin derivative, and a sugar alcohol.

Preferably the chitin and/or chitin derivative is selected from the group consisting of alpha (α) and beta (β) chitin, derivatives thereof as well as polymorphic forms, and chitosan and derivatives thereof.

More preferred, the chitin and/or chitin derivative is selected from chitosan modified with a metal silicate (such as calcium silicate, magnesium silicate or aluminum silicate) or a substituted chitosan (such as chitosan succinate, chitosan maleate, chitosan salysilate salts and their amide analogues).

Preferably, the sugar alcohol is selected from a group consisting of mannitol, sorbitol, isomaltose, maltitol, lactitol, erythritol, arabitol, xylitol, ribitol and/or mixtures thereof.

In one embodiment, the chitin and/or chitin derivative has a molecular weight of up to 2000 kD, preferably up to 1000 kD, preferably alpha and beta chitin.

It is also preferred that the chitin and/or chitin derivative has a mean particle size of less than 100 µm, preferably less than 50 µm, preferably less than 20 µm.

Moreover, it is preferred that the excipient for oral solid formulation comprises from 5 to 95, preferably 40-90, or even more preferably 70-90 weight percent (wt%) of chitin and/or chitin derivative, based on the weight of the excipient.

Additionally, the excipient for oral solid formulation preferably comprises from 5-95, preferably 5-50, more preferably 10-50 weight percent of sugar alcohol, based on the weight of excipient.

Moreover, it is preferred that the excipient for orally disintegrating solid formulation comprises sugar alcohol from 5 to 95, preferably 40-90, more preferably 60-90 weight percent of, based on the weight of the excipient.

Additionally, the excipient for orally disintegrating solid formulation preferably comprises from 5-95, preferably 5-50, more preferably 10-40 weight percent of chitin and/or chitin derivative, based on the weight of excipients.

It is also preferred that the excipient for orally disintegrating solid formulations comprises from 5 to 95, preferably 40-90, more preferably 60-90 weight percent, more preferably 70-90 weight percent of sugar alcohol, based on the weight of excipient.

Additionally, a pharmaceutical excipient may be preferred in a solid dosage form.

Most preferred, the excipient comprises one or more pharmacologically active ingredients such as methyldopa, amlodipine besylate, risperidone, bisoprolol fumarate, and atorvastatin calcium, enalapril maleate, phloroglucinol, mecobalamin, paracetamol.

The excipient according to the invention may be easily prepared by dry granulation or wet granulation, followed by drying and sizing the granules obtained.

The excipient may be preferably used for oral delivery of a pharmacologically active ingredient incorporated.

In one aspect of the present invention a method for preparing a pharmaceutical excipient for oral solid formulations is provided, wherein an aqueous solution of a sugar alcohol is sprayed onto chitin and/or chitin derivatives of 100% passed through mesh 20, followed by drying and sizing the excipient thus prepared.

In another aspect a method for preparing a pharmaceutical excipient is provided, wherein a mixture of sugar alcohol and chitin and/or chitin derivatives of 100% passed through mesh 20 is provided, followed by either wet granulation or dry compaction, drying and sizing the excipient thus prepared.

The object of the present invention is achieved by a pharmaceutical excipient according to claim 1, methods for preparing such a pharmaceutical excipient according to claims 13 and 14 and the use thereof according to claim 15. Preferred embodiments are disclosed in the sub claims.

The invention shall now be further described in the following example, without it being limited thereto.

### EXAMPLE 1

### Preparation of Mannitolized Chitin by Spray Granulation for Oral Solid Formulation:

Mannitol (20 g) was dissolved in water (100 ml) by stirring. Chitin (80 g) was placed in fluid-bed dryer. Then, the fluidized chitin was spray granulated with mannitol aqueous solution at a temperature of 50-60°C. The obtained granules were sized using a sieve with a suitable mesh.

### EXAMPLE 2

### Particle Size Distribution of Mannitolized Chitin for Oral Solid Formulation:

The prepared excipient of example 1 was tested for particle size distribution and the result is listed in the table below.

| Sieve no (mm) | % Retained | |
|---|---|---|
| | Chitin | Mannitolized Chitin |
| > 0.355 | ∼20 | ∼25 |
| 0.355 - 0.14 | ∼50 | ∼55 |
| < 0.14 | ∼30 | ∼20 |
| % Water | 8 | 6 |

### EXAMPLE 3

### Preparation for Oral Solid Formulation Containing 250 mg Methyldopa:

Methyldopa initially slugged and passed through mesh 22 (285 gm) was mixed with mannitolized chitin prepared according to example 1 (147.5 gm), magnesium stearate (2.5 mg) was added to the blend, mixed and compressed in tablets. The obtained tablets were then film coated.

### EXAMPLE 4

### Physical Properties of Methyldopa 250 mg Tablets:

The physical properties of the tablets prepared in example 3 are measured according to the USP 32 NF 27shown in table below.

| Product Name | Uncoated Tablet Weight (mg) | Hardness (N) | Friability (%) | Disintegration (min) |
|---|---|---|---|---|
| Tablets of example 3 | 435 | 140 | 0.25 | 0.3 (20 sec) |

The dissolution profile was performed in 0.1 M HCl (900 mL, apparatus II, 50 rpm)

| Product Name | % Release | | |
|---|---|---|---|
| | 5 min | 10 min | 15 min |
| Tablets of example 3 (film-coated) | 95 | 99 | 101 |
| Aldomet 250 film coated tablets | 92 | 101 | 102 |

### EXAMPLE 5

### Preparation for Oral Solid Formulation Containing 10 mg Amlodipine:

Amlodipine besylate (14 gm equivalent to 10 gm amlodipine base) was mixed with mannitolized chitin prepared according to example 1 (185 gm), magnesium stearate (1.0 mg) was added to the blend, mixed and compressed in tablets.

### EXAMPLE 6

### Physical Properties of Amlodipine 10 mg Tablets:

The physical properties of the tablets prepared in example 5 are shown in the table below.

| Product Name | Uncoated Tablet Weight (mg) | Hardness (N) | Friability (%) | Disintegration (min) |
|---|---|---|---|---|
| Tablets of example 5 | 200 | 110 | 0.15 | 0.8 (50 sec) |

The dissolution profile was performed in 0.01 M HCl (500 mL, apparatus II, 75 rpm)

| Product Name | % Release | | |
|---|---|---|---|
| | 10 min | 20 min | 30 min |
| Tablets of example 3 | 97 | 98 | 98 |
| Norvasc 5 capsules | 88 | 92 | 96 |

### EXAMPLE 7

### Preparation of Chitinized Mannitol by Wet Granulation for Orally Disintegrating Formulation:

A mixture of mannitol (70 g) and chitin (20 g) was dry mixed and granulated with water containing 10 g of mannitol. The obtained wet granules were dried and sized using a sieve with a suitable mesh.

### EXAMPLE 8

### Preparation of Chitinized Mannitol by Dry Granulation for Orally Disintegrating Formulation:

A mixture of mannitol (80 g) and chitin (20 g) was dry mixed and slugged by compactor or compression machine. The obtained solid was sized using a sieve with a suitable mesh.

### EXAMPLE 9

### Particle Size Distribution of Chitinized Mannitol:

The prepared excipients of examples 7 and 8 were tested for particle size distribution and the results are listed in the table below.

| Sieve no (mm) | % Retained | |
|---|---|---|
| | Example 7 | Example 8 |
| > 0.355 | ∼ 30 | ∼ 40 |
| 0.355 - 0.18 | ∼ 35 | ∼ 20 |
| < 0.18 | ∼ 35 | ∼ 40 |
| %Water | 1.5% | 1.5% |

### EXAMPLE 10

### Preparation of Orally Disintegrating Phloroglucinol 80 mg Tablets:

Phloroglucinol dihydrate (16.8 g) was mixed with chitinized mannitol prepared according to example 7 (79.2 g), aspartame (0.5 g), strawberry powder (2 g) and sodium stearyl fumarate (1.5 g) were sieved, added to the blend, mixed and compressed in tablets.

### EXAMPLE 11

### Preparation of Orally Disintegrating Mecobalamin 5 mg Tablets:

Mecobalamin (1.83 g) was mixed with chitinized mannitol prepared according to example 7 (94.17 g), aspartame (0.5 g), strawberry powder (2 g) and sodium stearyl fummarate (1.5 g) were sieved, added to the blend, mixed and compressed in tablets.

### EXAMPLE 12

### Preparation of Orally Disintegrating Enalapril Maleate 2.5 mg Tablets:

Enalapril maleate (0.85 g) was mixed with chitinized mannitol prepared according to example 7 (95.15 g), aspartame (0.5 g), strawberry powder (2 g) and sodium stearyl fummarate (1.5 g) were sieved, added to the blend, mixed and compressed in tablets.

### EXAMPLE 13

### Physical Properties of Prepared Tablets of Examples 10, 11 and 12 Using Chitinized Mannitol Prepared According to Example 7:

The physical properties of the tablets prepared in examples 10, 11 and 12 are shown in table below.

| Product Name | Tablet Weight (mg) | Hardness (N) | Friability (%) | Disintegration (sec) | Wetting Time (sec)* |
|---|---|---|---|---|---|
| Tablets of example 10 | 500 | 50 - 60 | 0.5 | 12 | 13 |
| Tablets of example 11 | 300 | 60 - 70 | 0.5 | 14 | 15 |
| Tablets of example 12 | 300 | 50 - 60 | 0.5 | 11 | 19 |

| | | | | | |
|---|---|---|---|---|---|
| *Wetting time is obtained by using a piece of double folded tissue paper placed in a petridish containing 6 ml of water. One tablet was placed on this paper and the time for complete wetting of tablet was noted as wetting time | | | | | |

### EXAMPLE 13

### Preparation of Orally Disintegrating Phloroglucinol 80 mg Tablets:

Phloroglucinol dihydrate (16.8 g) was mixed with chitinized mannitol prepared according to example 8 (79.2 g), aspartame (0.5 g), strawberry powder (2 g) and sodium stearyl fummarate (1.5 g) were sieved, added to the blend, mixed and compressed in tablets.

### EXAMPLE 14

### Preparation of Orally Disintegrating Mecobalamin 5 mg Tablets:

Mecobalamin (1.83 g) was mixed with chitinized mannitol prepared according to example 8 (94.17 g), aspartame (0.5 g), strawberry powder (2 g) and sodium stearyl fummarate (1.5 g) were sieved, added to the blend, mixed and compressed in tablets.

### EXAMPLE 15

### Preparation of Enalapril Maleate 2.5 mg Tablets:

Enalapril maleate (0.85 g) was mixed with chitinized mannitol prepared according to example 8 (95.15 g), aspartame (0.5 g), strawberry powder (2 g) and sodium stearyl fummarate (1.5 g) were sieved, added to the blend, mixed and compressed in tablets.

### EXAMPLE 16

### Physical Properties of Prepared Tablets of Examples 13, 14 and 15 Using Chitinized Mannitol Prepared According to Example 8:

The physical properties of the tablets prepared in examples 13 to 15 are shown in table below.

| Product Name | Tablet Weight (mg) | Hardness (N) | Friability (%) | Disintegration (sec) | Wetting Time (sec) |
|---|---|---|---|---|---|
| Tablets of example 13 | 500 | 75 - 105 | 0.2 | 22 | 22 |
| Tablets of example 14 | 300 | 50-70 | 0.6 | 15 | 13 |
| Tablets of example 15 | 300 | 50-75 | 0.3 | 16 | 24 |

The features disclosed in the foregoing description and the claims may, both separately and in any combination thereof, be material for realizing the invention in diverse forms thereof.

## Claims

1. Pharmaceutical excipient comprising chitin and/or a chitin derivative, and a sugar alcohol.

2. Pharmaceutical excipient according to claim 1, wherein the chitin and/or chitin derivative is selected from the group consisting of alpha and beta chitin and derivatives thereof and polymorphic forms, and chitosan and derivatives thereof.

3. Pharmaceutical excipient according to claim 2, wherein the chitin and/or chitin derivative is selected from chitosan modified with metal silicate, such as calcium silicate, magnesium silicate and aluminum silicate, substituted chitosan, such as chitosan succinate, chitosan maleate, chitosan salysilate salts and their amide analogues.

4. Pharmaceutical excipient according to any of the preceding claims wherein the sugar alcohol is selected from a group consisting of mannitol, sorbitol, isomaltose, maltitol, lactitol, erythritol, arabitol, xylitol, ribitol and mixtures thereof.

5. Pharmaceutical excipient according to any of the preceding claims, wherein the chitin and/or chitin derivative has a molecular weight of up to 2000 kD, preferably up to 1000 kD, preferably alpha and beta chitin.

6. Pharmaceutical excipient for orally disintegrating solid formulation according to any of the preceding claims, wherein the chitin and/or chitin derivative has a mean particle size of less than 100 µm, preferably less than 50 µm, preferably less than 20 µm.

7. Pharmaceutical excipient according to any of the preceding claims, wherein the excipient for oral solid formulations comprises from 5 to 95, preferably 40-90, more preferably 70-90 weight percent of chitin and/or chitin derivative, based on the weight of the excipient.

8. Pharmaceutical excipient according to any of the preceding claims, wherein the excipient for oral solid formulations comprises from 5-95, preferably 5-50, more preferably 10-50 weight percent of sugar alcohol, based on the weight of excipient.

9. Pharmaceutical excipient according to any of the preceding claims, wherein the excipient for orally disintegrating solid formulations comprises from 5-95, preferably 5-50, more preferably 10-40 weight percent, more preferably 10-30 weight percent of chitin and/or chitin derivative, based on the weight of the excipient.

10. Pharmaceutical excipient according to any of the preceding claims, wherein the excipient for orally disintegrating solid formulations comprises from 5 to 95, preferably 40-90, more preferably 60-90 weight percent, more preferably 70-90 weight percent of sugar alcohol, based on the weight of excipient.

11. Pharmaceutical excipient according to any of the preceding claims, wherein it is in a solid dosage form.

12. Pharmaceutical excipient according to any of the preceding claims, wherein it comprises one or more pharmacologically active ingredients selected from the group consisting of methyldopa, amlodipine besylate, risperidone, bisoprolol fumarate, atorvastatin calcium, phloroglucinol, mecobalamin, and enalapril maleate.

13. Method for preparing a pharmaceutical excipient for oral solid formulations, wherein an aqueous solution of a sugar alcohol is sprayed onto chitin and/or chitin derivatives of 100% passed through mesh 20, followed by drying and sizing the excipient thus prepared.

14. Method for preparing a pharmaceutical excipient, wherein a mixture of sugar alcohol and chitin and/or chitin derivatives of 100% passed through mesh 20 is provided, followed by either wet granulation or dry compaction, drying and sizing the excipient thus prepared.

15. Use of a pharmaceutical excipient according to any of the claims 1 to 12 for oral delivery of a pharmacologically active ingredient incorporated.

## Amended claims

### Amended claims in accordance with Rule 137(2) EPC.

**1.** Method for preparing a pharmaceutical excipient for oral solid formulations comprising chitin and a sugar alcohol, wherein either an aqueous solution of the sugar alcohol is sprayed onto the chitin, or a mixture of the sugar alcohol and the chitin is provided followed by wet granulation, wherein 100 % of chitin passed through mesh 20, followed by drying and sizing the excipient thus prepared.

**2.** Method according to claim 1, **characterized in that** the chitin is selected from the group consisting of alpha- and beta-chitin.

**3.** Method according to claim 1 or 2, **characterized in that** the chitin is alpha-chitin.

**4.** Method according to any of the preceding claims, **characterized in that** the sugar alcohol is selected from a group consisting of mannitol, sorbitol, isomaltose, maltitol, lactitol, erythritol, arabitol, xylitol, ribitol, and mixtures thereon.

**5.** Method according to claim 4, **characterized in that** the sugar alcohol is mannitol.

**6.** Method according to any of the preceding claims, **characterized in that** the chitin has a molecular weight of up to 2000 kD, preferably up to 1000 kD.

**7.** Method according to any of the preceding claims, **characterized in that** the chitin is alpha-chitin having a mean particle size of less than 100 μm, preferably less than 50 μm, preferably less than 20 μm.

**8.** Method according to any of the preceding claims, **characterized in that** the excipient for oral solid formulations comprises from 5 to 95, preferably 40-90, more preferably 70-90 weight percent of alpha-chitin, based on the weight of the excipient.

**9.** Method according to any of the preceding claims, **characterized in that** the excipient for oral solid formulations comprises from 5-95, preferably 5-50, more preferably 10-50 weight percent of mannitol, based on the weight of the excipient.

**10.** The process according to claims 1 to 7, **characterized in that** the excipient for orally disintegrating solid formulations comprises from 5-95, preferably 5-50, more preferably 10-30 weight percent of alpha-chitin, based on the weight of the excipient.

**11.** Method according to claims 1 to 7, and 10, wherein the excipient for orally disintegrating solid formulations comprises from 5 to 95, preferably 40-90, more preferably 70-90 weight percent of mannitol, based on the weight of the excipient.

**12.** Method according to any of the preceding claims, **characterized in that** it comprises one or more pharmacologically active ingredients selected from the group consisting of methyldopa, amlodipine besylate, risperidone, bisoprolol fumarate, atorvastatin calcium, phloroglucinol, mecobalamin, enalapril maleate and paracetamol.
